# EUROPEAN PATENT APPLICATION

(11) **EP 4 692 322 A1**
(43) Date of publication of application: **11.02.2026**
(21) Application number: 24785182.7
(22) Date of filing: 02.04.2024
(51) Int. Cl.: C12N 5/077, C12N 5/00

(54) **COMPOSITION CONTAINING EXTRACELLULAR MATRIX AND MATRIX-BOUND VESICLES FOR PRODUCING EXTRACELLULAR MATRIX AND PREPARATION METHOD THEREFOR**

(30) Priority: 03.04.2023 KR 20230043837
(71) Applicant: HLB Cell Co., Ltd, Hwaseong-si, Gyeonggi-do 18469 (KR)
(72) Inventor: YOON, Hee-Hoon, Yongin-si Gyeonggi-do 16836 (KR); KIM, Yu-Mi, Suwon-si Gyeonggi-do 16507 (KR); KIM, Jeong-Jun, Pyeongtaek-si Gyeonggi-do 17920 (KR); LEE, Ji-Hye, Suwon-si Gyeonggi-do 16491 (KR)
(74) Representative: J A Kemp LLP
(86) International application number: PCT/KR2024/004270
(87) International publication number: WO 2024/210477

(57) **Abstract**

The disclosure relates to a method for preparing an extracellular matrix through the cultivation of connective tissue cells, and a use of the prepared extracellular matrix. More specifically, the disclosure relates to a method for culturing connective tissue cells using a serum-free and xenogeneic component-free culture medium, and can be effectively utilized to increase the production of extracellular matrix. Additionally, the extracellular matrix prepared by the preparation method of the disclosure is derived from human cells without the use of xenogeneic components such as fetal bovine serum, etc., and thus can be safely and effectively used in the development of medical devices for tissue therapy, including cell therapy products and medical supplies, or cosmetic raw materials.

## Description

### Technical Field

This application claims priority and the benefit of Korean Patent Application No. 10-2023-0043837 filed in the Korean Intellectual Property Office on 3 April 2023, the disclosure of which is incorporated herein by reference.

The disclosure relates to a method for extracellular matrix production through the culture of connective tissue cells and use of the produced extracellular matrix and, more specifically, to a culture medium capable of producing the extracellular matrix through the co-culture of connective tissue cells with epithelial (tissue) cells or through the culture of connective tissue cells without co-culture with epithelial cells and a culture method therefor, and to a composition prepared by the method and containing extracellular matrix and matrix-bound nanovesicles.

### Background Art

Extracellular matrix (ECM), which is a collection of biopolymers that fill gaps between cells to physically hold tissues, belongs to the connective tissue of mammals and is abundant in bone, teeth, tendons, and skin. The extracellular matrix supports the body, connects tissues, participates in the regeneration of damaged tissues, and regulates various physiological functions, such as cell division, proliferation, migration, and differentiation. Examples of components constituting the extracellular matrix include collagen, laminin, fibronectin, polysaccharides, and glycoproteins.

Particularly, the extracellular matrix is becoming increasingly important in the fields of cell therapy and regenerative medicine employing embryonic stem cells or adult stem cells, and tissue engineering, which aims to fabricate tissues ex vivo by culturing cells within extracellular matrix-mimicking biomaterials.

In modern medicine, damaged or dysfunctional tissues can be replaced with healthy tissues through allogeneic transplantation. However, healthy allogeneic tissues are limited in supply, and thus another form of tissue supply is needed. Organoid techniques enable the formation of tissues having the same gene from tiny amounts of biological samples collected from patients, for the purpose of tissue transplantation. Currently, during organoid production, Matrigel (Corning's product) or similar products are used as cell culture scaffolds for three-dimensional cell culture. Matrigel is a basement membrane extracellular matrix composition consisting of Type IV collagen, laminin, entactin, and the like. However, this material is derived from mouse sarcoma, and is strictly used for research purposes due to the risks of infection, heterogeneous immune response, and unidentified tumorigenic components.

Therefore, there is a need for a novel method of producing extracellular matrix at a high concentration without using mouse sarcoma-derived cells. Particularly, for the use of extracellular matrix in the fields of organoids and three-dimensional culture, it is necessary to develop a method for producing an extracellular matrix in a self-assembling form.

Throughout the specification, many papers and patent documents are referenced and their citations are provided. The disclosures of cited papers and patent documents are entirely incorporated by reference herein, and the level of skill in the art to which the disclosure pertains and details of the disclosure are explained more clearly.

### Disclosure of Invention

### Technical Problem

The present inventors made intensive efforts to solve the problems with respect to conventional methods for producing extracellular matrix. As a result, the present inventors identified that the culture of connective tissue cells in a serum-free/xeno-free culture medium can be effectively utilized for increasing the amount of extracellular matrix produced, and thus completed the disclosure.

An aspect of the disclosure is to provide a composition for extracellular matrix production containing an extracellular matrix and matrix-bound nanovesicles.

Another aspect of the disclosure is to provide a method for preparing a composition for extracellular matrix production containing an extracellular matrix and matrix-bound nanovesicles.

Other purposes and advantages of the disclosure will become more obvious when taken with the following detailed description of the invention, claims, and drawings.

### Solution to Problem

According to an aspect of the disclosure, there is provided a composition for extracellular matrix production containing an extracellular matrix and matrix-bound nanovesicles.

The present inventors established that the amount of extracellular matrix produced was excellent when human-derived connective tissue cells were cultured in a serum-free/xeno-free culture medium, and identified that the presence of the extracellular matrix and matrix-bound nanovesicles inhibit self-assembly of the extracellular matrix.

As used herein, the term matrix-bound nanovesicles (MBV) refers to a type of nanovesicles that bind to the matrix. The matrix-bound nanovesicles have been reported to alter the genes, proteins, and cell-surface marker expression patterns of macrophages to suppress inflammatory responses and enhance pro-remodeling (Crum RJ, et al. Immunomodulatory matrix-bound nanovesicles mitigate acute and chronic pristane-induced rheumatoid arthritis. NPJ Regen Med. 2022 Feb 2;7(1):13).

In an embodiment of the disclosure, the composition may be obtained by culturing connective tissue cells or connective tissue cells and epithelial tissue cells in a culture medium.

In one embodiment of the disclosure, the culture medium may be a serum-free culture medium.

As used herein, the term "serum-free" means that the serum derived from humans or animals are not substantially contained. Particularly, the meaning that the serum derived from humans or animals are not substantially contained is that the serum derived from humans or animals is contained at a content lower than that in conventional media, or at a content that does not substantially affect cell proliferation or survival. Specifically, the term means that the serum derived from humans or animals is contained at a content of 1 wt% or less.

In one embodiment of the disclosure, the culture medium may be a xeno-free culture medium.

As used herein, the term "xeno-free" means that other components derived from humans or animals are not substantially contained. Particularly, the meaning that "other components derived from animals are not substantially contained" encompasses "being substantially free of animal proteins", and means that other animal-derived products or compounds are absent or substantially absent. The term "animals" refers to mammals, birds, reptiles, fish, insects, spiders, or other animal species, excluding humans, and does not encompass microorganisms, such as bacteria and cells.

Additionally, the meaning that other components derived from animals are not substantially contained is that other components derived from animals are contained at a content lower than that in conventional media, or at a content that does not substantially affect cell proliferation or survival. Specifically, the term means that other components derived from animals are contained at a content of 1 wt% or less.

In an embodiment of the disclosure, the culture medium may contain at least one selected from the group consisting of a glucocorticoid, insulin, and a growth factor.

In an embodiment of the disclosure, the growth factor may be at least one selected from the group consisting of fibroblast growth factor (FGF), keratinocyte growth factor (KGF), insulin-like growth factor (IGF), nerve growth factor (NGF), platelet-derived growth factor (PDGF), transforming growth factor-β (TGF-β), and vascular endothelial growth factor (VEGF), but is not limited thereto.

In an embodiment of the disclosure, the ErbB signaling growth factor may be at least one selected from the group consisting of epidermal growth factor (EGF), transforming growth factor-a (TGF-α), heregulin-β (HRG-β), heparin-binding EGF-like growth factor, amphiregulin, betacellulin, epiregulin, epigen, and neuregulins, but is not limited thereto.

In an embodiment of the disclosure, the matrix-bound nanovesicles may inhibit self-assembly of the extracellular matrix.

In an embodiment of the disclosure, the self-assembly may include fibrillation, network forming, or a combination thereof of the extracellular matrix.

In an embodiment of the disclosure, the extracellular matrix may contain at least one selected from the group consisting of laminin, collagen, fibronectin, proteoglycan, entactin, and fibulin, but is not limited thereto.

In an embodiment of the disclosure, the self-assembly of the extracellular matrix does not occur since the matrix-bound nanovesicles inhibit the self-assembly of the extracellular matrix in the composition containing the extracellular matrix and the matrix-bound nanovesicles.

In accordance with another aspect of the present invention, there is provided a method for extracellular matrix production, the method including:
(a) culturing connective tissue cells in a culture medium containing at least one selected from the group consisting of a glucocorticoid, insulin, and a growth factor to obtain a culture or a cell layer; and
(b) collecting an extracellular matrix and matrix-bound nanovesicles from the culture or cell layer in step (a).

In an embodiment of the disclosure, the matrix-bound nanovesicles may be bound to the extracellular matrix.

The present inventors established that the amount of extracellular matrix produced was excellent when human-derived connective tissue cells were cultured in a serum-free/xeno-free culture medium.

In an embodiment of the disclosure, the extracellular matrix may contain at least one selected from the group consisting of laminin, collagen, fibronectin, proteoglycan, entactin, and fibulin, but is not limited thereto.

The following steps included in an embodiment of the disclosure will be described in more detail.

### (a) Step of culturing connective tissue cells in a culture medium containing at least one selected from the group consisting of a glucocorticoid, insulin, and a growth factor to obtain a culture or a cell layer

In one embodiment of the disclosure, the connective tissue cells may be human-derived cells. The human-derived cells may be normal cells or genetically modified cells.

In an embodiment of the disclosure, the connective tissue cells may be at least one selected from the group consisting of fibroblasts, osteocytes, adipocytes, chondrocytes, ligament cells, tendon cells, mesenchymal stem cells, and cancer-associated fibroblasts, but are not limited thereto.

In an embodiment, the extracellular matrix may contain at least one selected from the group consisting of laminin, collagen, fibronectin, proteoglycan, entactin, and fibulin, but is not limited thereto.

In one embodiment of the disclosure, the culture medium may be a serum-free culture medium.

In one embodiment of the disclosure, the culture medium may be a xeno-free culture medium.

A basal medium of the culture medium according to the disclosure may be any basal medium that is used for animal cell culture in the art.

In an embodiment of the disclosure, the basal medium of the culture medium is Dulbecco's Modified Eagle's Media (DMEM) .

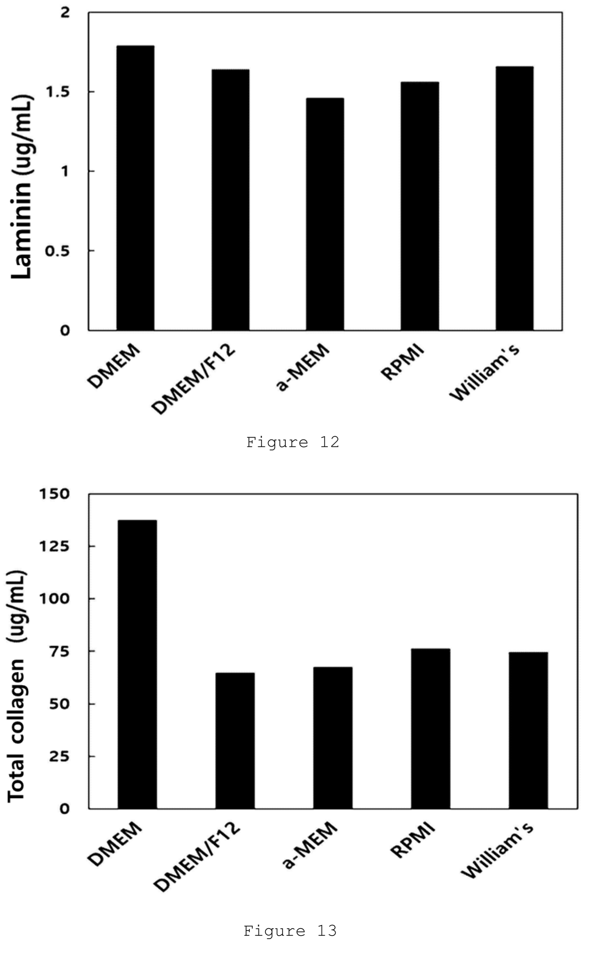

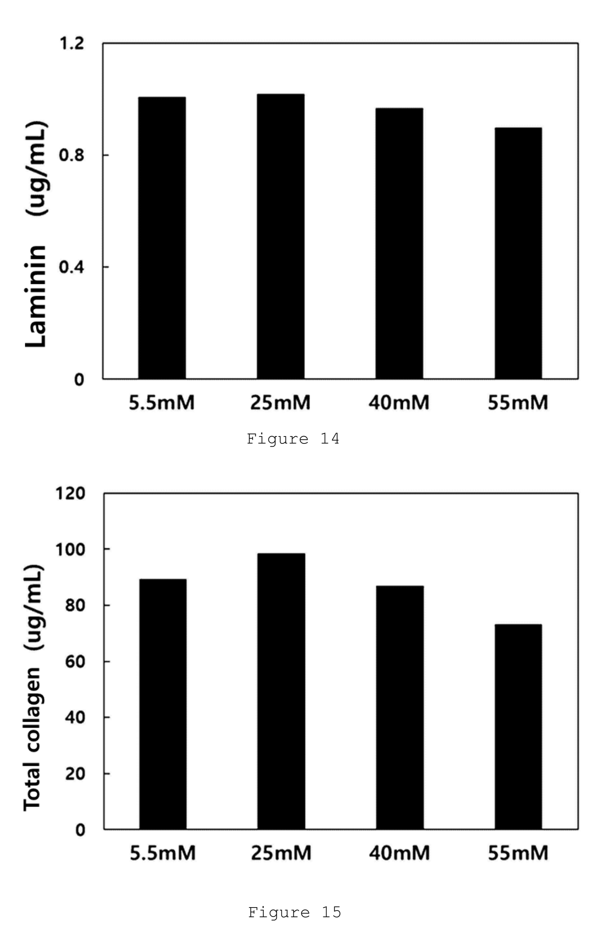

According to an exemplary embodiment of the disclosure, Dulbecco's Modified Eagle's Medium (HG-DMEM), Dulbecco's Modified Eagle Medium:Ham's F-12 (1:1) (DMEM/F12), α-Modification Minimum Essential Medium Eagle(α-MEM), RPMI1640, and Williams Media were individually used as basal media, and then the laminin and total collagen contents were analyzed. The amount of extracellular matrix produced were high when DMEM was used as a basal medium (FIGS. 12 and 13).

In an embodiment of the disclosure, the glucocorticoid may be at least one selected from the group consisting of dexamethasone, hydrocortisone, prednisone, prednisolone, methylprednisolone, betamethasone, triamcinolone acetonide, fludrocortisones, and cortisol.

In an embodiment of the disclosure, the ErbB signaling growth factor may be at least one selected from the group consisting of epidermal growth factor (EGF), transforming growth factor-a (TGF-α), heregulin-β (HRG-β), heparin-binding EGF-like growth factor, amphiregulin, betacellulin, epiregulin, epigen, and neuregulins, but is not limited thereto.

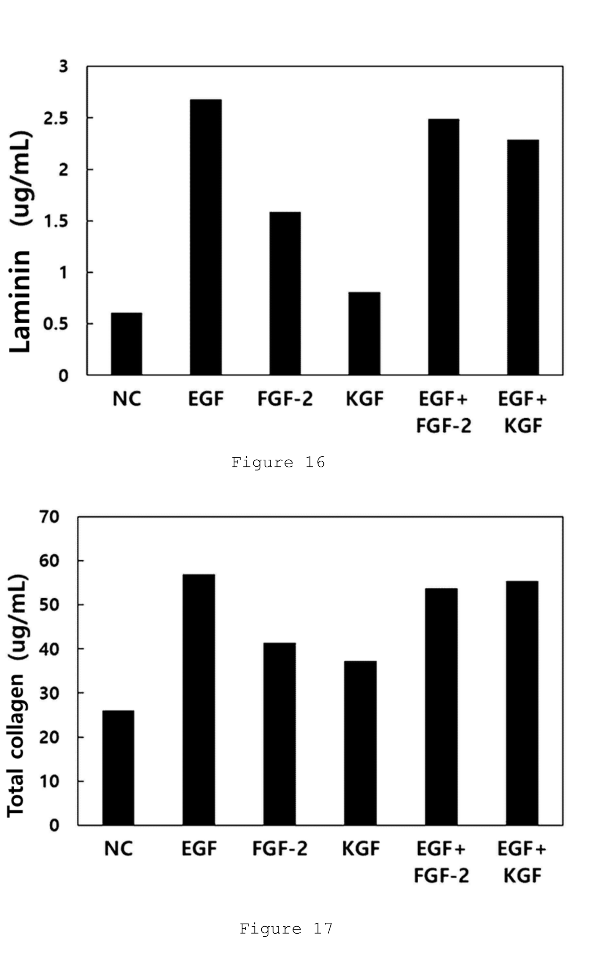

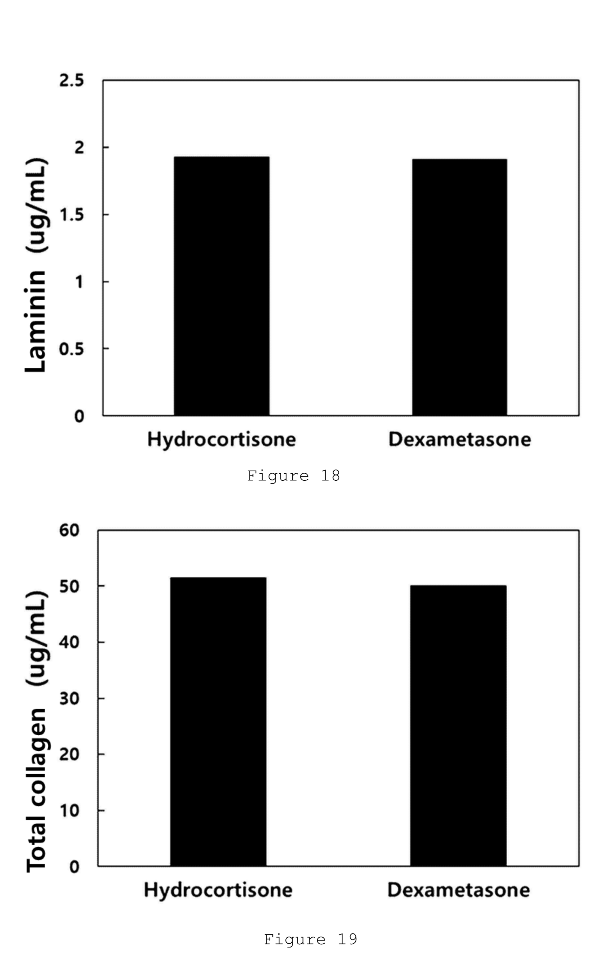

According to an exemplary embodiment of the disclosure, when the epidermal growth factor was used as a growth factor, the laminin and total collagen concentrations were highest, indicating that a highest amount of extracellular matrix was produced (FIGS. 16 and 17).

In an embodiment of the disclosure, the culture medium may further contain glucose.

In an embodiment of the disclosure, the glucose concentration is 1 mM to 55 mM.

In an exemplary embodiment of the disclosure, the amount of extracellular matrix produced was highest when the glucose concentration in the culture medium was 1 mM to 55 mM (FIGS. 14 and 15). The glucose concentration in the culture medium may be specifically, 1 mM to 55 mM, 1 mM to 50 mM, 1 mM to 45 mM, 1 mM to 40 mM, 1 mM to 35 mM, 1 mM to 30 mM, 1 mM to 25 mM, 2 mM to 55 mM, 2 mM to 50 mM, 2 mM to 45 mM, 2 mM to 40 mM, 2 mM to 35 mM, 2 mM to 30 mM, 2 mM to 25 mM, 5.5 mM to 55 mM, 5.5 mM to 50 mM, 5.5 mM to 45 mM, 5.5 mM to 40 mM, 5.5 mM to 35 mM, 5.5 mM to 30 mM, 5.5 mM to 5.55 mM, 10 mM to 55 mM, 10 mM to 50 mM, 10 mM to 45 mM, 10 mM to 40 mM, 10 mM to 35 mM, 10 mM to 30 mM, 10 mM to 25 mM, 15 mM to 55 mM, 15 mM to 50 mM, 15 mM to 45 mM, 15 mM to 40 mM, 15 mM to 35 mM, 15 mM to 30 mM, 15 mM to 25 mM, 20 mM to 55 mM, 20 mM to 50 mM, 20 mM to 45 mM, 20 mM to 40 mM, 20 mM to 35 mM, or 20 mM to 30 mM, and more specifically 20 mM to 25 mM.

In an embodiment of the preset invention, the culturing of cells may be performed using a culture medium containing an antioxidant.

In an embodiment of the disclosure, the antioxidant may be at least one selected from the group consisting of vitamin A, vitamin C, vitamin E, selenium, coenzyme Q10, catechin, N-acetylcysteine (NAC), glutathione, beta-carotene, lycopene, lutein, polyphenols, cysteine, and taurine, but is not limited thereto.

In an embodiment of the preset invention, the culturing of cells may be performed using a culture containing an animal-derived product at a content of less than 1 wt%.

As used herein, the meaning that the "culture containing an animal-derived product at a content of less than 1 wt%" is "being free of animal-derived products" or "being substantially free from of animal-derived products". The meaning of "being free of animal-derived products" or "being substantially free from of animal-derived products" encompasses a meaning of "being free of animal proteins" or "being substantially free of animal proteins", and means that blood-derived, blood pooled, and other animal-derived products or compounds are absent or substantially absent. The term "animals" refers to mammals, birds, reptiles, fish, insects, spiders, or other animal species, excluding humans. The "animals" does not include microorganism, such as bacteria and cells. For example, a method excluding animal-derived products or a method substantially excluding animal-derived products indicates a method where animal-derived proteins, such as immunoglobulins, meat digests, meat by-products, and milk or dairy products or digests, are substantially absent, essentially absent, or completely absent. Therefore, examples of the method excluding animal-derived products include methods excluding meat and dairy products, or meat or dairy by-products (such as bacterial or cell culture methods or bacterial fermentation methods). The meaning of "completely absent" is that, within the detection range of an equipment or method used, a substance cannot be detected or the presence thereof cannot be confirmed. The meaning of "essentially absent" is that only a trace amount of substance may be detected.

In an embodiment of the disclosure, the culturing of cells may be performed by at least one culture method selected from the group consisting of hypoxic culture, batch culture, fed-batch culture, and continuous culture, but is not limited thereto.

The hypoxic culture of the disclosure indicates culture under a hypoxic condition with an oxygen partial pressure of 1% to 10%. More specifically, the oxygen partial pressure in the hypoxic culture may be 1% to 10%, 1% to 8%, 1% to 6%, 1% to 5%, 1% to 4%, 1% to 3%, 1% to 2%, 2% to 10%, 2% to 8%, 2% to 6%, 2% to 5%, 2% to 4%, 2% to 3%, 3% to 10%, 3% to 8%, 3% to 6%, 3% to 5%, 3% to 4%, 5% to 10%, 5% to 8%, 5% to 6%, 10%, 9%, 8%, 7%, 6%, 5%, 4%, 3%, 2%, or 1%, but is not limited thereto.

The batch culture of the disclosure indicates culture of cells for 7 days to 13 days without exchange of the culture.

The fed-batch culture of the disclosure indicates culture of cells for 7 days to 13 days without exchange of the culture while adding 10% to 30% of a culture daily.

The continuous culture of the disclosure indicates culture of cells for 13 days with exchange of 25% to 75% of a culture every two or three days.

In an embodiment of the disclosure, the culture medium further contains at least one selected from the group consisting of ascorbate-2-phosphate, isoprenaline, and triiodothyronine.

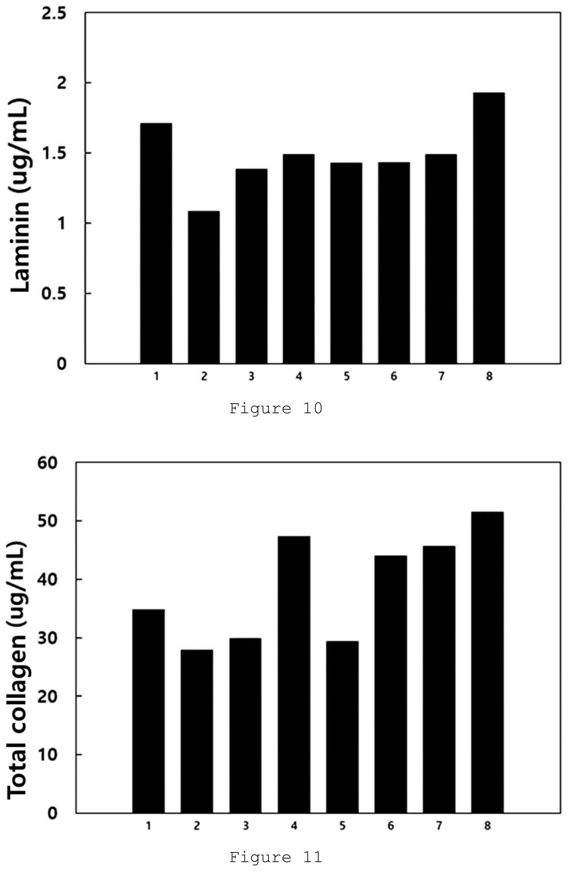

In an exemplary embodiment of the disclosure, the amount of extracellular matrix produced was high when all of ascorbate-2-phosphate, isoprenaline, and triiodothyronine were added to the culture medium (FIGS. 10 and 11).

### (b) Step of collecting an extracellular matrix and matrix-bound nanovesicles from the culture or cell layer in step (a)

In an embodiment of the disclosure, the culture may be obtained upon the exchange of the culture medium.

In an embodiment of the disclosure, the exchange of the culture medium may be performed at intervals of 1 to 10 days, specifically, 1 to 8 days, 1 to 6 days, 1 to 4 days, 1 to 3 days, 2 to 20 days, 2 to 8 days, 2 to 6 days, or 2 to 4 days, and more specifically, 2 to 3 days.

In an embodiment of the disclosure, the cell layer may be obtained after completion of culture.

### Advantageous Effects of Invention

The disclosure is directed to a method for extracellular matrix production through culture of connective tissue cells and to use of the produced extracellular matrix, and the method according to the disclosure employing a serum-free/xeno-free culture medium through the co-culture with or without epithelial (tissue) cells can be used to effectively produce the extracellular matrix. Furthermore, the extracellular matrix obtained by the production method of the disclosure is a human cell-derived extracellular matrix with the complete absence of a xeno-component such as fetal bovine serum, and thus can be safely and helpfully used in the future for the development of medical devices or cosmetic raw materials for tissue therapy, such as cell therapeutics and medical supplies.

Furthermore, the extracellular matrix obtained by the production method of the disclosure was identified to contain matrix-bound nanovesicles, and thus can be helpfully used in the future for the production of self-assembly-inhibited extracellular matrix.

### Brief Description of Drawings

FIG. 1 is a process diagram for extracellular matrix production through fibroblast single culture or fibroblast/keratinocyte co-culture.
FIG. 2 is a graph illustrating the results of analyzing the amount of laminin produced in a co-cultured or single cultured cell culture (*p < 0.05).
FIG. 3 is a graph illustrating the results of analyzing the amount of total collagen produced in a co-cultured or single cultured cell culture (*p < 0.05).
FIG. 4 is a graph illustrating the results of analyzing the amount of laminin produced in a cell culture or cell layer after single culture using a serum-free/xeno-free culture medium.
FIG. 5 is a graph illustrating the results of analyzing the amount of total collagen produced in a cell culture or cell layer after single culture using a serum-free/xeno-free culture medium.
FIG. 6 is a graph illustrating the results of analyzing the amounts of laminin produced in cell cultures after single culture of various types of connective tissue cells using a serum-free/xeno-free culture medium.
FIG. 7 is a graph illustrating the results of analyzing the amounts of total collagen produced in cell cultures after single culture of various types of connective tissue cells using a serum-free/xeno-free culture medium.
FIG. 8 is a graph illustrating the results of analyzing the amount of laminin produced in a cell culture depending on the additive composition (insulin, epidermal growth factor, hydrocortisone, ascorbate-2-phosphate, triiodothyronine, and isoprenaline) in a serum-free/xeno-free culture medium.
FIG. 9 is a graph illustrating the results of analyzing the amount of total collagen produced in a cell culture depending on the additive composition (insulin, epidermal growth factor, hydrocortisone, ascorbate-2-phosphate, triiodothyronine, and isoprenaline) in a serum-free/xeno-free culture medium.
FIG. 10 is a graph illustrating the results of analyzing the amount of laminin produced in a cell culture depending on the additive composition (ascorbate-2-phosphate, triiodothyronine, and isoprenaline) in a serum-free/xeno-free culture medium.
FIG. 11 is a graph illustrating the results of analyzing the amount of total collagen produced in a cell culture depending on the additive composition (ascorbate-2-phosphate, triiodothyronine, and isoprenaline) in a serum-free/xeno-free culture medium.
FIG. 12 is a graph illustrating the results of analyzing the amount of laminin produced in a cell culture depending on the basal medium of a serum-free/xeno-free culture medium.
FIG. 13 is a graph illustrating the results of analyzing the amount of total collagen produced in a cell culture depending on the basal medium of a serum-free/xeno-free culture medium.
FIG. 14 is a graph illustrating the results of analyzing the amount of laminin produced in a cell culture depending on the glucose concentration in a serum-free/xeno-free culture medium.
FIG. 15 is a graph illustrating the results of analyzing the amount of total collagen produced in a cell culture depending on the glucose concentration in a serum-free/xeno-free culture medium.
FIG. 16 is a graph illustrating the results of analyzing the amount of laminin produced in a cell culture depending on the type of growth factor in a serum-free/xeno-free culture medium.
FIG. 17 is a graph illustrating the results of analyzing the amount of total collagen produced in a cell culture depending on the type of growth factor in a serum-free/xeno-free culture medium.
FIG. 18 is a graph illustrating the results of analyzing the amount of laminin produced in a cell culture depending on the type of glucocorticoid in a serum-free/xeno-free culture medium.
FIG. 19 is a graph illustrating the results of analyzing the amount of total collagen produced in a cell culture depending on the type of glucocorticoid in a serum-free/xeno-free culture medium.
FIG. 20 is a graph illustrating the results of analyzing the amount of laminin produced in a cell culture depending on the type of ErbB signaling factor in a serum-free/xeno-free culture medium.
FIG. 21 is a graph illustrating the results of analyzing the amount of total collagen produced in a cell culture depending on the type of ErbB signaling factor in a serum-free/xeno-free culture medium.
FIG. 22 is a graph illustrating the results of analyzing the amount of laminin produced in a cell culture when a serum medium or a serum-free/xeno-free culture medium was used (*p < 0.05).
FIG. 23 is a graph illustrating the results of analyzing the amount of total collagen produced in a cell culture when a serum medium or a serum-free/xeno-free culture medium was used (*p < 0.05)
FIG. 24 is a graph illustrating the results of analyzing the amount of total collagen produced in a culture medium of single- or co-cultured cells according to an example of the disclosure (*p < 0.05).
FIG. 25 is a graph illustrating the results of analyzing the content of Type IV collagen secreted into a culture medium or deposited on a culture dish during co-culture (*p < 0.05).
FIGS. 26 is a graph illustrating the results of analyzing the content of laminin secreted into a culture medium or deposited on a culture dish during co-culture (*p < 0.05).
FIGS. 27 is a graph illustrating the results of analyzing the amount of total collagen produced in a culture medium after the administration of the antioxidant N-Acetylcysteine (NAC) to co-cultured cells (*p < 0.05).
FIG. 28 is a graph illustrating the results of analyzing the amount of Type IV collagen produced in a culture medium after the administration of the antioxidant vitamin C to co-cultured cells.
FIG. 29 is a graph illustrating the results of analyzing the amounts of laminin and Type IV collagen produced in a culture medium of cells co-cultured under a hypoxic condition (*p < 0.05).
FIG. 30 is a graph illustrating the results of analyzing the amounts of laminin and Type IV collagen produced in a culture medium of cells co-cultured by a batch culture method (*p < 0.05).
FIG. 31 is a graph illustrating the results of analyzing the amounts of laminin produced in a culture medium of cells co-cultured by a fed-batch culture method (*p < 0.05).
FIG. 32 is a graph illustrating the results of analyzing the amounts of laminin produced in a culture medium of cells co-cultured by a continuous culture method (*p < 0.05).
FIG. 33 is a graph illustrating the results of analyzing the type of extracellular matrix components contained in a cell culture when a serum-free/xeno-free culture medium was used.
FIG. 34 presents images showing the presence or absence of extracellular matrix self-assembly, and matrix-bound nanovesicles, confirmed when the extracellular matrix was observed through an optical microscope and a scanning electron microscope.

### Best Mode for Carrying out the Invention

Hereinafter, the disclosure will be described in more detail with reference to exemplary embodiments. These exemplary embodiments are provided only for the purpose of specifically illustrating the disclosure, and therefore, according to the purpose of the disclosure, it would be apparent to a person skilled in the art that these exemplary embodiments are not construed to limit the scope of the disclosure.

### Examples

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Experimental methods - Analysis of contents of extracellular matrix components in culture

### Analysis of total collagen content

To measure the human collagen content in the extracellular matrix present in a culture obtained according to each example described below, quantitative analysis was conducted by utilizing a commercially available Sirius Red kit (Chondrex, #9062). The analysis method followed the manufacturer's instructions. Specifically, Sirius Red solution was added to the culture, followed by incubation, and the precipitated collagen was then mixed with an acidic solution and quantitatively analyzed by measurement of absorbance at 530 nm.

### Analysis of laminin content

To measure the amount of human laminin component in the extracellular matrix present in a culture obtained according to each example described below, quantitative analysis was conducted by using a commercially available analysis kit (TakaRa, MK107). The analysis method followed the manufacturer's instructions. Specifically, the culture was added onto a 96-well plate coated with an anti-human laminin antibody, followed by incubation. Thereafter, residues were washed off, and a chromogenic enzyme-conjugated specific antibody was added, followed by incubation. Then, quantitative analysis was conducted by measurement of the absorbance at 450 nm.

### Example 1: Cell culture for extracellular matrix production

### Example 1.1 - Extracellular matrix production through single culture of human fibroblasts

### Example 1.1.1 - Culture of human fibroblasts

Human fibroblasts were seeded at a density of 3×10⁵ cells per 150-mm² culture dish. The cells were cultured in high-glucose Dulbecco's Modified Eagle's Medium (DMEM) supplemented with 10% fetal bovine serum and 1% penicillin for 7 days while a growth medium was exchanged every 2-3 days.

### Example 1.1.2 - Extracellular matrix production using serum-free/xeno-free medium

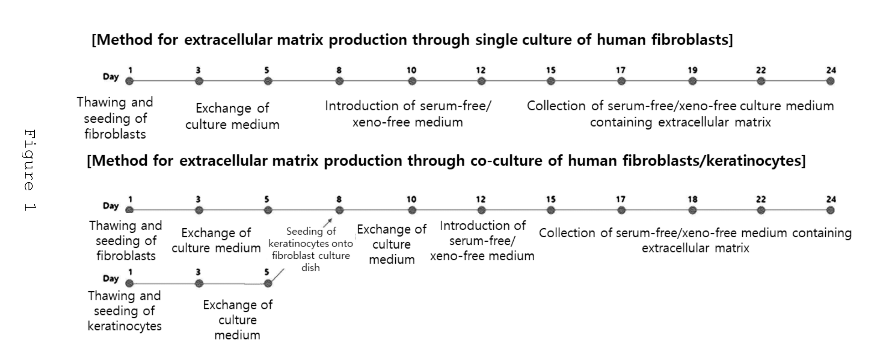

After being cultured according to Example 1.1.1, the cells were further cultured from day 8 using a serum-free/xeno-free culture medium [Dulbecco's Modified Eagle Medium:Ham's F-12 (1:1) (DMEM/F12) supplemented with 100 U/mL penicillin/streptomycin, 2 mM L-alanyl-L-glutamine, 5 µg/L selenium, 50 µM ascorbate-2-phosphate, 10 mg/L insulin, 1 µM hydrocortisone, 0.02 nM triiodothyronine, 1 µM isoprenaline, and 10 µg/L epidermal growth factor] while the medium was exchanged at intervals of 2 to 3 days. The cells were cultured for a total of 15 days. To wash off residual fetal bovine serum component, the cells were washed three times with Dulbecco's phosphate-buffered saline (D-PBS) prior to the exchange with the serum-free/xeno-free culture medium. Upon the medium exchange, the cell culture obtained by previous culture was collected and refrigerated. The process flow diagram is shown in FIG. 1. The cell culture after collection was analyzed according to Example 3. The composition of the serum-free/xeno-free culture medium capable of increasing the amount of extracellular matrix produced was optimized according to Example 2. Each culture was conducted under conditions of 37° C and 5% CO₂.

### Example 1.2 - Extracellular matrix production through co-culture of human fibroblasts and keratinocytes

### Example 1.2.1 - Culture of human fibroblasts

Human fibroblasts were seeded at a density of 3×10⁵ cells per 150-mm² culture dish. The cells were cultured in high-glucose Dulbecco's Modified Eagle's Medium (DMEM) supplemented with 10% fetal bovine serum and 1% penicillin for 7 days while a growth medium was exchanged every 2-3 days.

### Example 1.2.2 - Culture of human keratinocytes

Human keratinocytes were seeded at a density of 1.5×10⁶ cells per 175-mm² culture dish. The cells were cultured in keratinocyte serum-free medium (KSFM) supplemented with 5 ng/mL human recombinant epidermal growth factor and 50 µg/mL bovine pituitary extract (BPE) for 7 days while the medium was exchanged at intervals of 2 to 3 days.

### Example 1.2.3 - Extracellular matrix production through co-culture

The human keratinocyte culture obtained by culture for 7 days in Example 1.2.2 was removed, and the keratinocytes were suspended by treatment with chemical reaction. The human dermal fibroblasts culture obtained by culture for 7 days in Example 1.2.1 was removed, and then the keratinocytes were seeded thereon at a density of 1-2 × 10⁶ cells per culture dish and cultured in an incubator at 37°C for 48 hours. The human fibroblast/keratinocyte co-culture obtained by culture for 48 hours was removed, followed by washing once with Dulbecco's phosphate-buffered saline. After 35 mL of a serum-free/xeno-free culture medium was added, the cells were cultured for 13 days while the medium was exchanged at intervals of 2 to 3 days. The cell culture obtained by previous culture was collected and refrigerated. The contents of laminin and total collagen, which were produced from the cells single- or co-cultured by the above-described method and secreted into the culture medium, were analyzed.

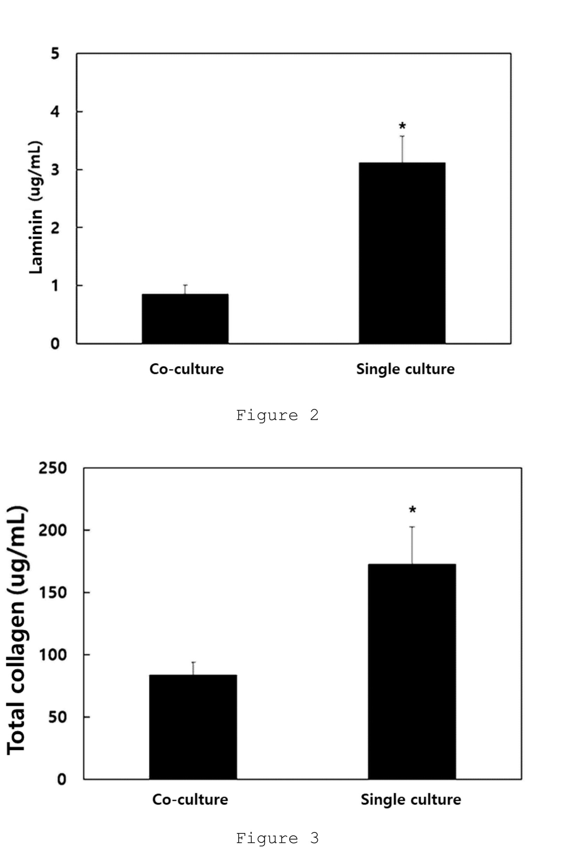

The results are shown in FIG. 2 and Table 3.

As shown in FIG. 2, 0.86 µg/mL laminin was produced for co-culture of fibroblasts and epithelial cells and 3.12 µg/mL laminin was produced for single culture of fibroblasts.

As shown in FIG. 3, 83.98 µg/mL total collagen was produced for co-culture of fibroblasts and epithelial cells and 173.05 µg/mL laminin was produced for single culture of fibroblasts.

The contents of laminin and total collagen, which were produced from the cells single cultured by the above-described method and secreted into the culture medium or deposited on the cell layer, were analyzed.

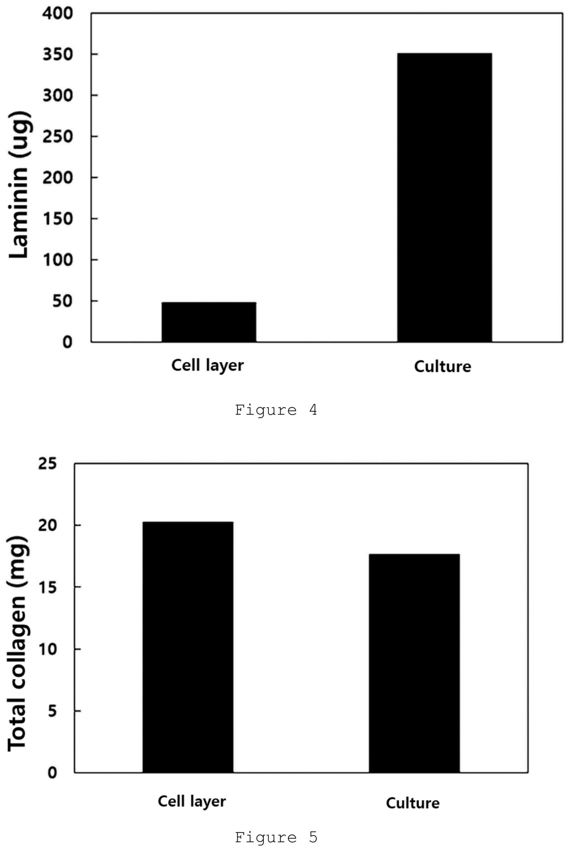

The results are shown in FIG. 4 and Table 5.

As shown in FIGS. 4 and 5, the amount of laminin secreted into the culture was 7.3-fold that on the cell layer, thus is higher than on the cell layer, whereas the amount of total collagen produced on the cell layer was similar to that in the culture.

### Example 1.3 - Extracellular matrix production using various types of connective tissue cells

### Example 1.3.1 - Culture of human connective tissue cells

Human fibroblasts, adipocytes, mesenchymal stem cells, and cancer-associated fibroblasts were seeded at a density of 3×10⁵ cells per 150-mm² culture dish. The cells were cultured in high-glucose Dulbecco's Modified Eagle's Medium (DMEM) supplemented with 10% fetal bovine serum and 1% penicillin for 7 days while the growth medium was exchanged every 2-3 days.

### Example 1.3.2 - Extracellular matrix production using culture of human connective tissue cells and serum-free/xeno-free medium

After being cultured according to Example 1.3.1, the cells were cultured from day 8 using a serum-free/xeno-free culture medium while the medium was exchanged at intervals of 2 to 3 days. The cells were cultured for a total of 15 days. To wash off residual fetal bovine serum component, the cells were washed three times with Dulbecco's phosphate-buffered saline (D-PBS) prior to the exchange with the serum-free/xeno-free culture medium. Upon the medium exchange, the cell culture obtained by previous culture was collected and refrigerated. The contents of laminin and total collagen, which were produced during culture of various types of connective tissue cells and secreted into the culture medium, were analyzed.

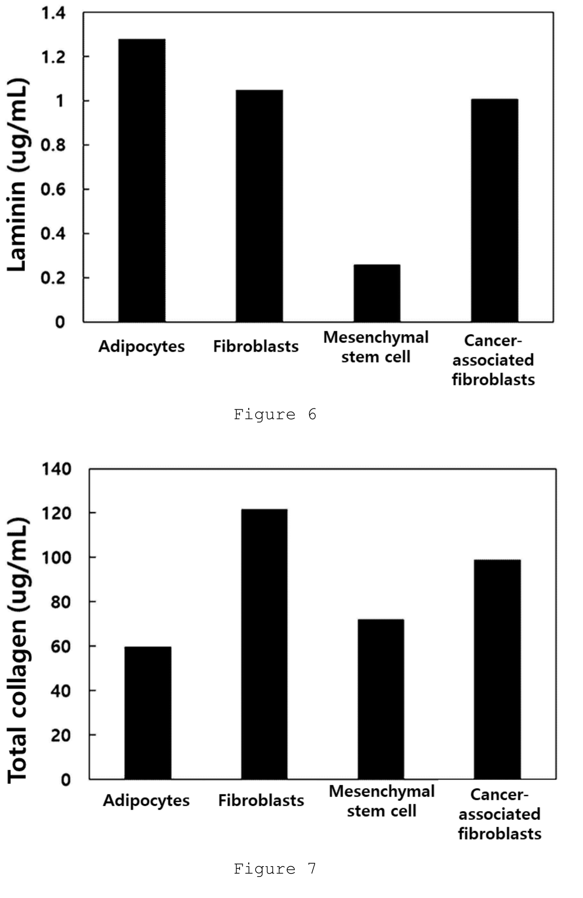

The results are shown in FIG. 6 and Table 7.

As shown in FIGS. 6 and 7, when a serum-free/xeno-free culture medium corresponding to the composition of the disclosure was applied, the extracellular matrix was produced through culture of various types of connective tissue cells (adipocytes, fibroblasts, mesenchymal stem cells, and cancer-associated fibroblasts).

### Example 2: Optimization of composition of serum-free/xeno-free culture medium for human fibroblast-derived extracellular matrix production

### Example 2.1 - Optimization of additive composition in serum-free/xeno-free culture medium

To discover factors that are expected to affect extracellular matrix production of fibroblasts, cell culture media with various compositions were prepared and applied to cell culture.

To compare the amount of extracellular matrix produced depending on the additive composition in a serum-free/xeno-free culture medium, Dulbecco's Modified Eagle Medium:Ham's F-12 (1:1) (DMEM/F12) supplemented with 100 U/mL penicillin/streptomycin (antibiotic for contamination prevention), 2 mM L-alanyl-L-glutamine (an amino acid supplement typically added to cell culture media), and 5 µg/L selenium (an antioxidant for long-term culture) was used as the basal medium. A medium containing all or none of 50 µM ascorbate-2-phosphate, 10 mg/L insulin, 1 µM hydrocortisone, 0.02 nM triiodothyronine, 1 µM isoprenaline, and 10 µg/L epidermal growth factor, expected to affect extracellular matrix production was selected as a control group. Culture media each lacking an individual factor were prepared, and then culture was performed according to Example 1.1. The composition of each serum-free/xeno-free culture medium used in the experiment is shown in Table 1 below.

**TABLE 1**

| Additive composition of used medium | | | | | | |
|---|---|---|---|---|---|---|
| NO | Insulin | Epidermal growth factor | Hydrocortisone | Ascorbate -2-phosphate | Triiodothyronine | Isoprenaline |
| 1 | X | ○ | ○ | ○ | ○ | ○ |
| 2 | ○ | X | ○ | ○ | ○ | ○ |
| 3 | ○ | ○ | X | ○ | ○ | ○ |
| 4 | ○ | ○ | ○ | X | ○ | ○ |
| 5 | ○ | ○ | ○ | ○ | X | ○ |
| 6 | ○ | ○ | ○ | ○ | ○ | X |
| 7 | ○ | ○ | ○ | ○ | ○ | ○ |
| 8 | X | X | X | X | X | X |

The laminin and total collagen concentrations in each cell culture obtained by culture according to the above-described method were compared and analyzed.

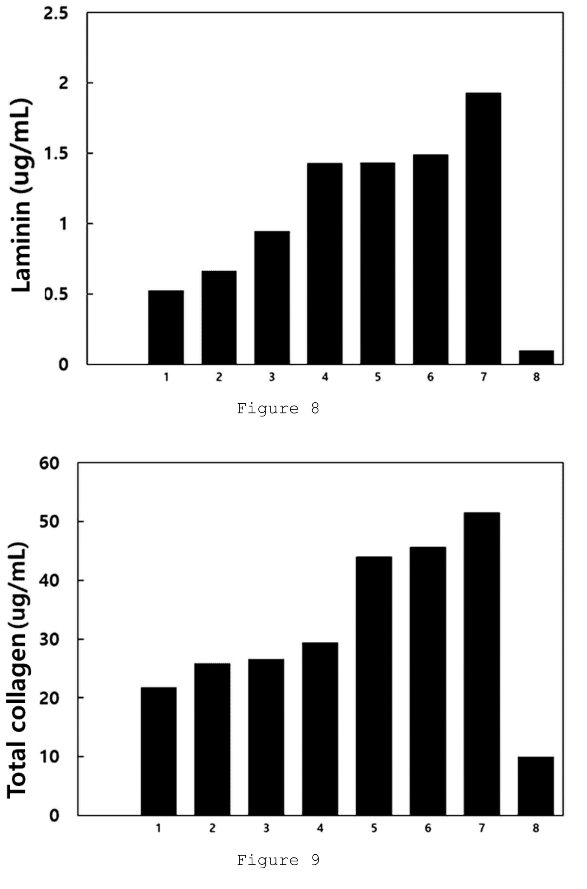

The results are shown in FIGS. 8 and 9.

As shown in FIGS. 8 and 9, the amount of extracellular matrix produced was significantly reduced when epidermal growth factor, insulin, or hydrocortisone was individually removed or all were removed, indicating that epidermal growth factor, insulin, or hydrocortisone is an essential factor in extracellular matrix production through single culture. Additionally, ascorbate-2-phosphate did not affect laminin production but greatly affected collagen production.

After the discovery of essential components, additional experiments for selecting additives affecting the amount of extracellular matrix produced were conducted. A medium containing insulin, 10 µg/L epidermal growth factor, and 1 µM hydrocortisone as essential components in addition to DMEM/F12 supplemented with 100 U/mL penicillin/streptomycin, 2 mM L-alanyl-L-glutamine, and 5 µg/L selenium was used as the basal medium, and at least one of 50 µM ascorbate-2-phosphate, 0.02 nM triiodothyronine, and 1 µM isoprenaline was added thereto, thereby preparing a culture medium. Then, culture was performed according to Example 1.1. The composition of each serum-free/xeno-free culture medium used in the experiment is shown in Table 2 below.

**TABLE 2**

| Additive composition of used medium | |
|---|---|
| NO | Factors added to media |
| 1 | - |
| 2 | 0.02 nM triiodothyronine |
| 3 | 1 µM isoprenaline |
| 4 | 50 µM ascorbate-2-phosphate |
| 5 | 1 µM isoprenaline + 0.02 nM triiodothyronine |
| 6 | 50 µM ascorbate-2-phosphate + 1 µM isoprenaline |
| 7 | 50 µM ascorbate-2-phosphate + 0.02 nM triiodothyronine |
| 8 | 50 µM ascorbate-2-phosphate + 0.02 nM triiodothyronine + 1 µM isoprenaline |

The laminin and total collagen concentrations in each cell culture obtained by culture using the individual composition in Table 2 were compared and analyzed.

The results are shown in FIGS. 10 and 11.

As shown in FIGS. 10 and 11, triiodothyronine and isoprenaline affected extracellular matrix production. Additionally, ascorbate-2-phosphate did not affect laminin production but greatly affected collagen production.

### Example 2.2 - Selection of basal medium for serum-free/xeno-free culture medium

To compare the amount of extracellular matrix produced depending on the basal medium for a serum-free/xeno-free culture medium in Example 1.1, the cells were cultured using Dulbecco's Modified Eagle's Medium (HG-DMEM), Dulbecco's Modified Eagle Medium:Ham's F-12 (1:1) (DMEM/F12), α-Modification Minimum Essential Medium Eagle(α-MEM), or RPMI1640, Williams Media, each of which is supplement with 100 U/mL penicillin/streptomycin, 2 mM L-alanyl-L-glutamine, 5 µg/L selenium, 10 mg/L insulin, 10 µg/L epidermal growth factor, 50 µM ascorbate-2-phosphate, 0.02 nM triiodothyronine, 1 µM isoprenaline and 1 µM hydrocortisone. The laminin and total collagen concentrations in each cell culture obtained by culture according to the above-described method were compared and analyzed.

The results are shown in FIGS. 12 and 13.

As shown in FIGS. 12 and 13, the laminin concentration did not differ among the basal media, whereas the total collagen concentration was produced at a highest concentration when DMEM was used as a basal medium.

### Example 2.3 - Optimization of glucose concentration in serum-free/xeno-free culture medium

To compare the amount of extracellular matrix produced depending on the glucose concentration in a serum-free/xeno-free culture medium used in Example 1.1, the cells were cultured in DMEM media with glucose concentrations of 5.5 mM, 25 mM, 40 mM, and 55 mM and then the total collagen and laminin contents in each cell culture were analyzed.

The results are shown in FIGS. 14 and 15.

As shown in FIGS. 14 and 15, cytotoxicity was induced due to a high concentration of glucose when the glucose concentration was 55 mM, and thus the amount of extracellular matrix produced was reduced.

### Example 2.4 - Optimization of type and concentration of growth factor in serum-free/xeno-free culture medium

To compare the amount of extracellular matrix produced depending on the type and concentration of growth factor in a medium, an epidermal growth factor, a fibroblast growth factor, or a keratinocyte growth factor was added, and the cells were cultured according to Example 1.1. The growth factors shown in Table 3 were added to media containing ascorbate-2-phosphate, insulin, selenium, hydrocortisone, triiodothyronine, and isoprenaline in basal media, and the cells were cultured according to Example 1.1.

**TABLE 3**

| Type and concentration of growth factors added to media | |
|---|---|
| NO | Growth factors added to media |
| 1 | - |
| 2 | 10 ng/mL epidermal growth factor |
| 3 | 10 ng/mL fibroblast growth factor-2 |
| 4 | 15 ng/mL keratinocyte growth factor |
| 5 | 10 ng/mL epidermal growth factor, 10 ng/mL fibroblast growth factor-2 |
| 6 | 10 ng/mL epidermal growth factor, 15 ng/mL keratinocyte growth factor |

The growth factors were added, and then the cells were cultured. Subsequently, the laminin and total collagen concentrations in a cell culture were compared and analyzed.

The results are shown in FIGS. 16 and 17.

As shown in FIGS. 16 and 17, the amounts of laminin and total collagen produced were increased in all the experimental groups with growth factors. Particularly, when the epidermal growth factor was added alone, the laminin concentration was 2.68 ug/mL and the total collagen concentration was 56.94 ug/mL, indicating a highest amount of extracellular matrix produced.

### Example 2.5 - Selection of glucocorticoid for serum-free/xeno-free culture medium

To compare the amount of extracellular matrix produced depending on the type of glucocorticoid in a medium, hydrocortisone as native glucocorticoid or dexamethasone as synthetic glucocorticoid was added, and the cells were cultured according to Example 1.1.

A culture medium containing 100 U/mL penicillin/streptomycin, 2 mM L-alanyl-L-glutamine, 5 µg/L selenium, 10 mg/L insulin, 10 µg/L epidermal growth factor, 50 µM ascorbate-2-phosphate, 0.02 nM triiodothyronine, and 1 µM isoprenaline in DMEM/F12 was used as a basal medium, and 1 µM hydrocortisone or 0.1 µM dexamethasone was added, and thereafter, the cells were cultured according to Example 1.1. The laminin and total collagen concentrations depending on the addition of hydrocortisone or dexamethasone were compared and analyzed.

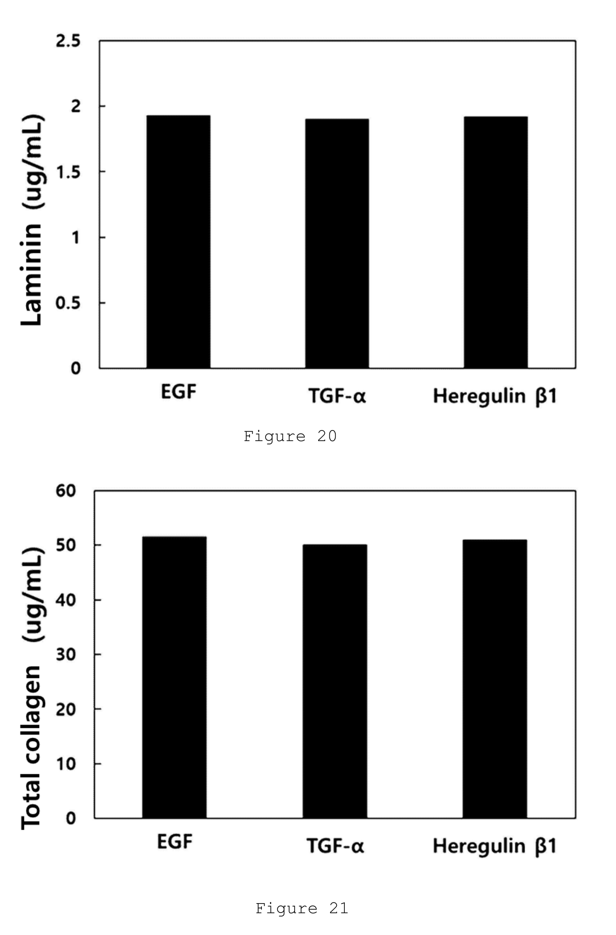

The results are shown in FIGS. 18 and 19.

As shown in FIGS. 18 and 19, the amount of extracellular matrix produced was not significantly different between two experimental groups.

### Example 2.6 - Selection of ErbB signaling factor in serum-free/xeno-free culture medium

To compare the amount of extracellular matrix produced depending on the type of ErbB signaling factor in a medium, epidermal growth factor (EGF), transforming growth factor-a (TGF-α), or heregulin-β1 was added, and the cells were cultured according to Example 1.1.

A culture medium containing 100 U/mL penicillin/streptomycin, 2 mM L-alanyl-L-glutamine, 5 µg/L selenium, 10 mg/L insulin, 50 µg/L ascorbate-2-phosphate, 0.02 nM triiodothyronine, 1 µM isoprenaline, and 1 µM hydrocortisone in DMEM/F12 was used as a basal medium, and 10 ug/L epidermal growth factor, 10 ug/L transforming growth factor-a, or 10 ug/L heregulin-β1 was individually added, and thereafter, the cells were cultured according to Example 1.1. The laminin and total collagen concentrations depending on the addition of epidermal growth factor, transforming growth factor-α, or heregulin-β1 were analyzed and compared.

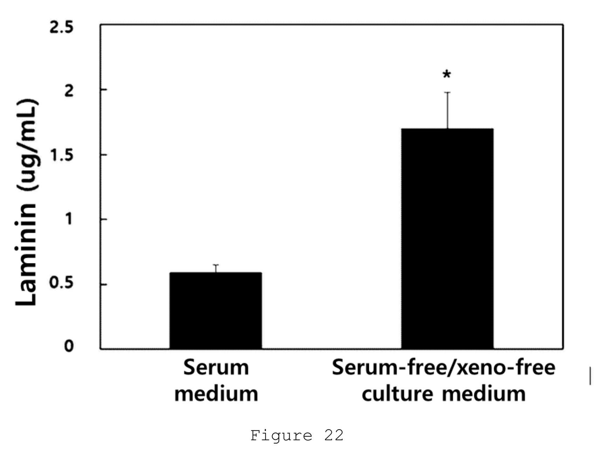

The results are shown in FIGS. 20 and 21.

As shown in FIGS. 20 and 21, the amount of extracellular matrix produced was not significantly different between two experimental groups.

### Example 2.7 - Comparison of amount of extracellular matrix produced in serum-free/xeno-free culture medium

### Example 2.7.1 - Production of extracellular matrix using single culture and serum medium

After being cultured according to Example 1.1.1, the cells were cultured from day 8 by the addition of high glucose Dulbecco's Modified Eagle's Medium (DMEM) supplement with 10% fetal bovine serum and 1% penicillin was added while the medium was exchanged every 2-3 days. The cells were cultured for a total of 15 days. Upon the medium exchange, the cell culture obtained by previous culture was collected and refrigerated. The cell culture, after collection, was analyzed according to Example 3, and each culture was conducted under conditions of 37°C and 5% CO₂.

### Example 2.7.2 - Production of extracellular matrix using serum-free/xeno-free medium

After being cultured according to Example 2.7.1, the cells were cultured from day 8 by addition of a serum-free/xeno-free medium while the medium was exchanged every 2-3 days. The cells were cultured for a total of 15 days. To wash off residual fetal bovine serum component, the cells were washed three times with Dulbecco's phosphate-buffered saline (D-PBS) prior to the exchange with the serum-free/xeno-free culture medium. Upon the medium exchange, the cell culture obtained by previous culture was collected and refrigerated. The cell culture after collection was analyzed according to Example 3, and each culture was conducted under conditions of 37°C and 5% CO₂. The contents of laminin and total collagen produced from the cells cultured using the serum medium and from the cells cultured using the serum-free/xeno-free medium were analyzed.

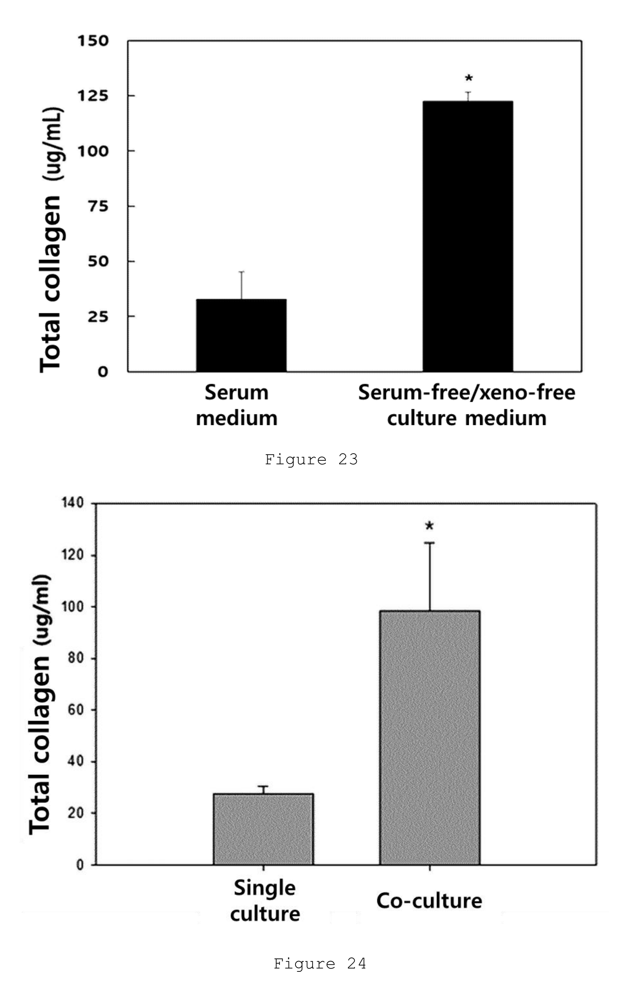

The results are shown in FIGS. 22 and 23.

As shown in FIGS. 22 and 23, the contents of laminin and total collagen when the cells were cultured by addition of a serum-free/xeno-free culture medium corresponding to the composition of the disclosure were 2.8-fold and 3.7-fold, respectively, compared with those when the cells were cultured by a culture method using a generally used serum medium, indicating a significant increase in the amount of extracellular matrix produced.

### Example 3: Cell culture for extracellular matrix production

### Example 3.1 - Production of extracellular matrix

### Example 3.1.1 - Culture of human fibroblasts

Frozen human dermal fibroblasts were thawed and seeded at a density of 3×10⁵ cells per 150-cm² culture dish. The cells were cultured using high-glucose Dulbecco's Modified Eagle's Medium (DMEM) supplemented with 10% fetal bovine serum and 1% penicillin while the medium was exchanged every 2-3 days. The cells were cultured for 7 days until the cells reached 100% confluence in the culture vessel.

### Example 3.1.2 - Culture of human keratinocyte

Frozen human keratinocytes were thawed and seeded at a density of 1.5×10⁶ cells per 175-cm² culture dish. The cells were cultured using Keratinocyte serum-free medium (KSFM) supplemented with 2.5 µg of human recombinant EGF and 25 mg of a bovine pituitary extract while the medium was exchanged every 2-3 days. The cells were cultured for 7 days until the cells reached 100% confluence in the culture vessel.

### Example 3.1.3 - Culture of human fibroblasts/keratinocytes

The human keratinocyte culture obtained by culture for 7 days according to Example 3.1.2 was removed, followed by washing once with PBS. Thereafter, 3 mL of Accutase was added, and then the keratinocytes were suspended by treatment with chemical reaction for 10 minutes in an incubator at 37°C. After 40 mL of PBS was added to the Accutase solution, the mixture was centrifuged at 800 rpm and 25°C for 5 minutes, and the supernatant was then removed. To the keratinocyte precipitate, FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor (EGF), and 1 µM isoproterenol) supplemented with 5% fatal bovine serum was added. The human dermal fibroblasts culture obtained by culture for 7 days in Example 3.1.1 was removed, and then the keratinocytes were dispensed thereon at a density of 1-2 × 10⁶ cells per culture dish and cultured in an incubator at 37°C for 24 hours.

### Example 3.1.4 - Culture of human fibroblasts/keratinocytes

The human fibroblast/keratinocyte culture obtained by culture for 24 hours according to Example 3.1.3 was removed, followed by washing once with PBS. The cells were cultured by addition of 35 mL of serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor (EGF), and 1 µM isoproterenol), and then the cells were cultured in an incubator at 37°C for a total of 13 days while the medium was exchanged every 2-3 days. The culture obtained by previous culture was collected and refrigerated.

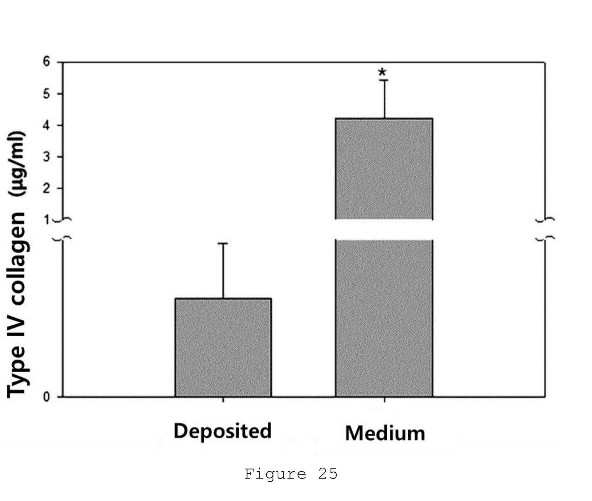

FIG. 24 illustrates the results of analyzing the content of total collagen, produced from the cells single-or co-cultured according to the above-mentioned and secreted into the culture medium. The single culture of fibroblasts produced 26-31 µg/mL collagen, and the co-culture of fibroblasts and epithelial cells produced 71-124 µg/mL collagen, corresponding to approximately 2.7-to 4-fold compared with that produced by the single culture.

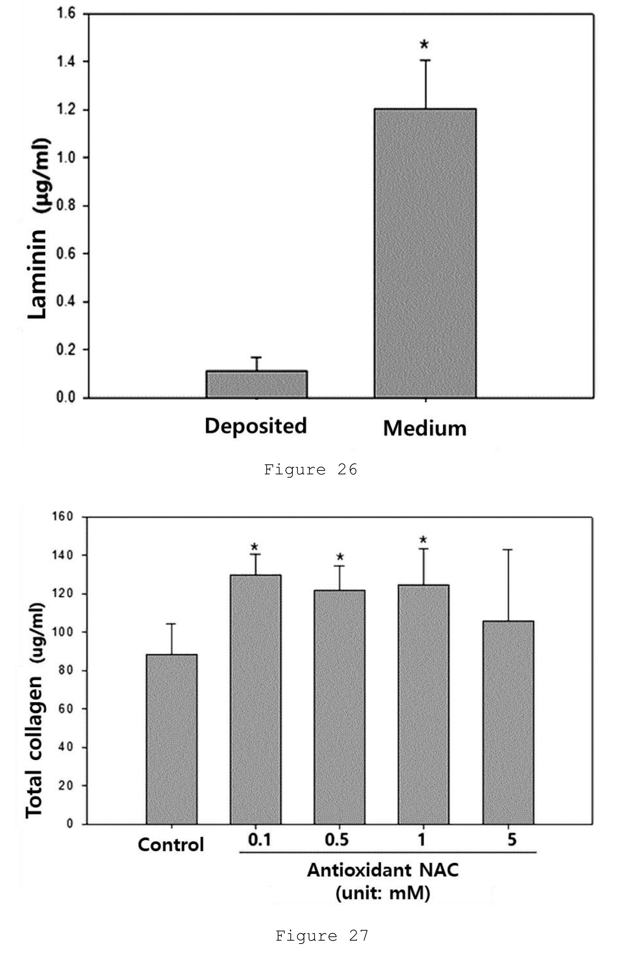

FIG. 25 illustrates the results of analyzing the content of Type IV collagen secreted into a culture medium or deposited on a culture dish during co-culture, and FIG. 26 illustrates the results of analyzing the content of laminin. Type IV collagen was secreted into the culture medium at approximately 67.4-fold that of the deposited amount, whereas laminin was secreted into the culture medium at approximately 6.18-fold that of the deposited amount.

### Example 4: Extracellular matrix production promoting effect using antioxidants

### Example 4.1 - Administration of N-acetyl cysteine (NAC)

The human fibroblast/keratinocyte culture obtained by culture for 24 hours according to Example 3.1.3 was removed, followed by washing once with PBS. To 35 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor (EGF), and 1 µM isoproterenol), N-acetyl cysteine (NAC) was added at a concentration of 0.1 mM, 0.5 mM, 1 mM, or 5 mM, and then the cells were cultured in an incubator at 37°C for a total of 13 days while the medium was exchanged every 2-3 days. The culture obtained by previous culture was collected.

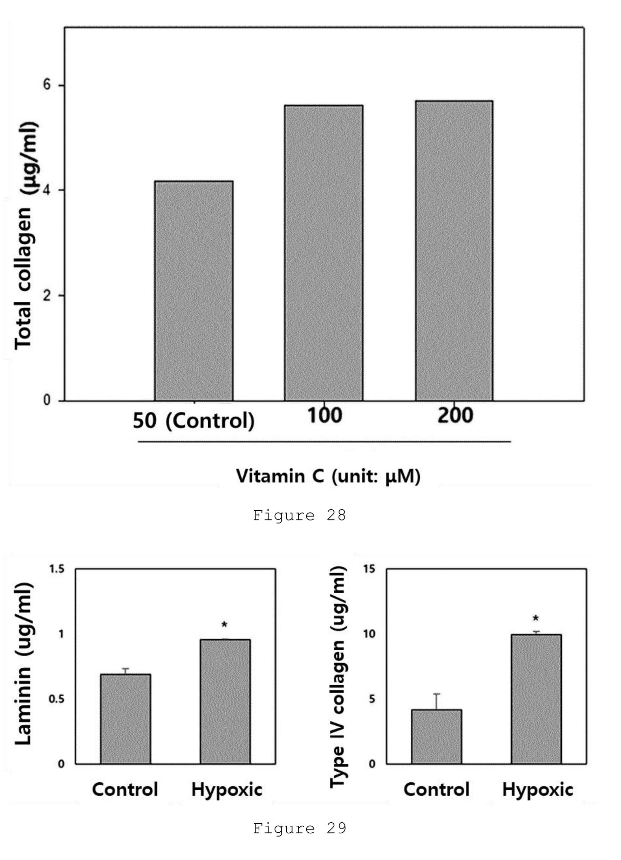

FIG. 27 illustrates the results of analyzing the content of total collagen produced after the administration of NAC to the cells obtained by culture according to the example. The collagen was produced at 77.1-99.5 µg/mL in a control group not administered NAC, 122.1-137.5 µg/mL in an experimental group administered 0.1 mM NAC, and 112.7-130.9 µg/mL in an experimental group administered 0.5 mM NAC. When NAC was administered at a concentration of 1 mM or higher, the amount of collagen produced was reduced and cytotoxicity was observed.

### Example 4.2 - Administration of vitamin C

The human fibroblast/keratinocyte culture obtained by culture for 24 hours according to Example 3.1.3 was removed, followed by washing once with PBS. To 35 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor (EGF), and 1 µM isoproterenol), vitamin C was added at a concentration of 50 µM, 100 µM, or 200 µM, and then the cells were cultured in an incubator at 37°C for a total of 13 days while the medium was exchanged every 2-3 days. The culture obtained by previous culture was collected.

Fig. 28 illustrates the results of analyzing the content of Type IV collagen produced after the administration of Vitamin C to the cells co-cultured according to the above-described example. Type IV collagen was produced at 4.17 µg/mL in a control group administered 50 µM vitamin C, 5.6 µg/mL in an experimental group administered 100 µM vitamin C, and 5.69 µg/mL in an experimental group administered 200 µM vitamin C.

### Example 5: Improvement of xeno-free culture process for reduction of extracellular matrix production cost

### Example 5.1 - Hypoxic culture

The human fibroblast/keratinocyte culture obtained by culture for 24 hours according to Example 3.1.3 was removed, followed by washing once with PBS. After 35 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor(EGF), and 1 µM isoproterenol), the cells were cultured under a hypoxic (5% oxygen) condition in an incubator at 37°C for a total of 13 days while the medium was exchanged every 2-3 days. The culture obtained by previous culture was collected.

FIG. 29 illustrates the results of comparative analysis with (the control group) in "an experiment where cells were cultured under an atmospheric oxygen concentration (21%) condition for a total of 13 days while culture exchange was conducted at intervals of 2-3 days" according to the above-described example. In the control group, 0.69 µg/mL laminin and 4.17 µg/mL Type IV collagen were produced, whereas in "the experiment group where cells were cultured under a hypoxic (5% oxygen) condition for a total of 13 days while culture exchange was conducted at intervals of 2-3 days", laminin was produced at 1.0 µg/mL, corresponding to approximately 1.5-fold that in the control group, and Type IV collagen was produced at 10.0 µg/mL, corresponding to approximately 2.4-fold that in the control group.

### Example 5.2 - Batch culture

The human fibroblast/keratinocyte culture obtained by culture for 24 hours according to Example 3.1.3 was removed, followed by washing once with PBS. After 35 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor(EGF), and 1 µM isoproterenol) was added, the cells were cultured in an incubator at 37°C for a total of 13 days without culture exchange. Thereafter, the culture obtained by previous culture was collected and refrigerated.

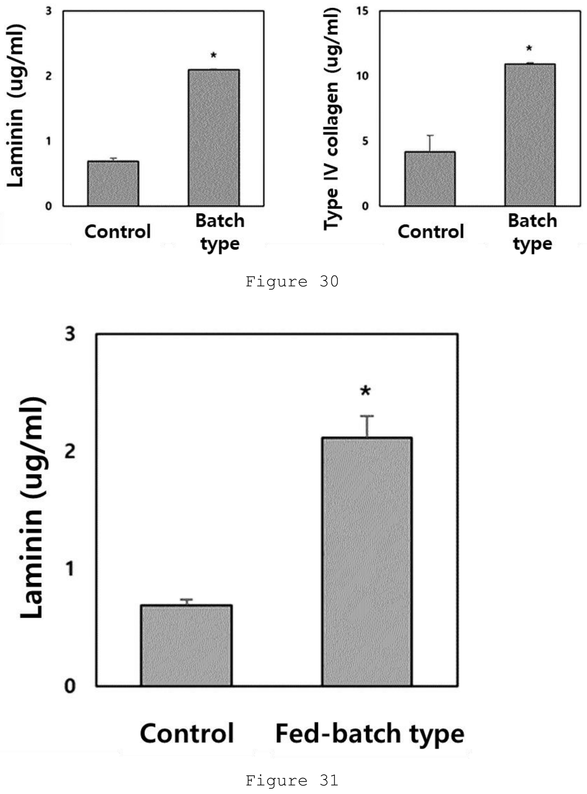

FIG. 30 illustrates the results of comparative analysis with (the control group) "in an experiment where cells were cultured for a total of 13 days while culture exchange was conducted at intervals of 2-3 days" according to the above-described example. In the control group, 0.69 µg/mL laminin and 4.17 µg/mL Type IV collagen were produced, whereas in "the experiment group where cells were cultured without culture exchange for a total of 13 days" (batch type), laminin was produced at 2.1 µg/mL, corresponding to approximately 3.0-fold that in the control group, and Type IV collagen was produced at 10.9 µg/mL, corresponding to approximately 2.6-fold that in the control group.

### Example 5.3 - Fed-batch culture

The human fibroblast/keratinocyte culture obtained by culture for 24 hours according to Example 3.1.3 was removed, followed by washing once with PBS. After 20 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor(EGF), and 1 µM isoproterenol) was added, the cells were cultured in an incubator at 37°C for a total of 8 days while the culture was added at 10% (2 mL/day) at intervals of 1 day. Thereafter, the culture obtained by previous culture was collected and refrigerated.

FIG. 31 illustrates the results of comparative analysis with (the control group) "in an experiment where cells were cultured for a total of 13 days while culture exchange was conducted at intervals of 2-3 days" according to the above-described example. In the control group, 0.69 µg/mL laminin was produced, whereas in "the experiment group where cells were cultured for a total of 8 days while the culture was added at 10% (2 mL/day) at intervals of 1 day" (fed-batch type), laminin was produced at 2.12 µg/mL, corresponding to approximately 3.1-fold that in the control group.

### Example 5.4 - Continuous culture

The human fibroblast/keratinocyte culture obtained by culture for 24 hours according to Example 3.1.3 was removed, followed by washing once with PBS. After 35 mL of a serum-free FAD medium (DMEM/F12 (1:1) + Glutamax-I, 1% penicillin, 2 mM L-glutamine, 10 mg/L insulin, 5.5 mg/L transferrin, 5 µg/L selenite, 50 µM L-ascorbic acid 2-phosphate, 1 µM hydrocortisone, 0.02 nM triiodothyronine (T3), 10 µg/L epidermal growth factor(EGF), and 1 µM isoproterenol) was added, the cells were cultured in an incubator at 37°C for a total of 13 days while a part (50%) of the culture was exchanged every 2-3 days. The culture collected was refrigerated.

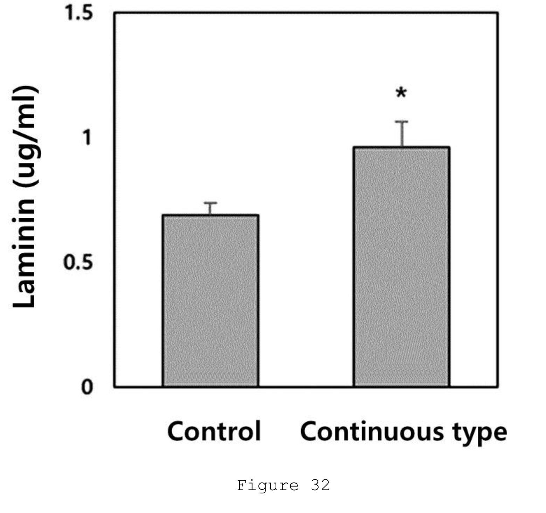

FIG. 32 illustrates the results of comparative analysis with (the control group) "in an experiment where cells were cultured for a total of 13 days while the entire culture was exchanged at intervals of 2-3 days" according to the above-described example. In the control group, 0.69 µg/mL laminin was produced, whereas in "the experiment group where cells were cultured for a total of 13 days while a portion (50%) of the culture was exchanged at intervals of 2-3 days" (continuous type), laminin was produced at 0.96 µg/mL, corresponding to approximately 1.4-fold that in the control group.

### Example 6: Analysis of contents of extracellular matrix components in culture

### Example 6.1 - Analysis of human collagen content

To measure the human collagen content in the extracellular matrix present in a culture obtained according to the example, quantitative analysis was conducted by utilizing a commercially available Sirius Red kit (Chondrex, #9062). The analysis method followed the manufacturer's instructions. Specifically, Sirius Red solution was added to the culture, followed by incubation, and the precipitated collagen was then mixed with an acidic solution, and quantitatively analyzed by measurement of absorbance at 530 nm.

### Example 6.2 - Analysis of human Type IV collagen content

To measure the amount of human Type IV collagen in the extracellular matrix present in a culture obtained according to the example, quantitative analysis was conducted by using a commercially available analysis kit (Aviva Systems Biology, OKCD06075). The analysis method followed the manufacturer's instructions. Specifically, the culture was added onto a 96-well plate coated with anti-human Type IV collagen antibody, followed by incubation. Thereafter, residues were washed off, and a chromogenic enzyme-conjugated specific antibody was added, followed by incubation. Then, quantitative analysis was conducted by measurement of the absorbance at 450 nm.

### Example 6.3 - Analysis of human laminin content

To measure the amount of human laminin component in the extracellular matrix present in a culture obtained according to the example, quantitative analysis was conducted by using a commercially available analysis kit (TaKaRa, MK107). The analysis method followed the manufacturer's instructions. Specifically, the culture was added onto a 96-well plate coated with anti-human laminin antibody, followed by incubation. Thereafter, residues were washed off, and a chromogenic enzyme-conjugated specific antibody was added, followed by incubation. Then, quantitative analysis was conducted by measurement of the absorbance at 450 nm.

The analysis results of contents of human collagen, human Type IV collagen, and human laminin according to Example 6 are shown in FIGS. 24 to 32.

### Example 7: Analysis of serum-free/xeno-free culture medium

### Example 7.1 - Analysis of extracellular matrix using LC-MS/MS

To quantitatively analyze the types of extracellular matrix components contained in the obtained extracellular matrix, LC-MS/MS quantitative analysis was performed.

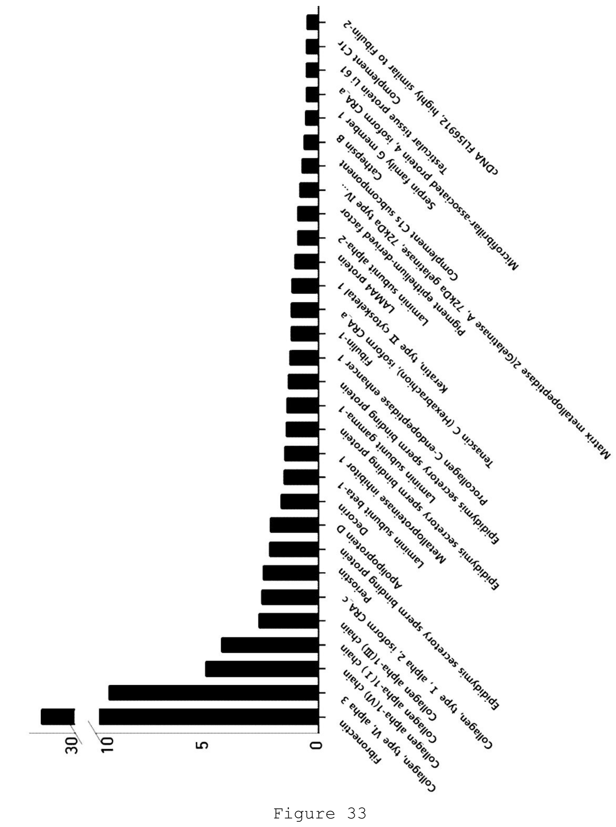

The obtained extracellular matrix was separated according to the protein molecular weight by using sodium dodecyl sulfate polyacrylamide gel electrophoresis (SDS-PAGE), and the separated proteins were visualized by Coomassie blue (InstantBlue^{®}) staining, and then divided into quarters according to the molecular weight. Thereafter, the proteins were subjected to cysteine alkylation and reduction, and then digested with trypsin at 37°C for 16 hours. The resultant peptides after digestion were extracted with 80% acetonitrile, completely dried in a vacuum concentrator, and then reconstituted in 0.1% formic acid, thereby obtaining peptide samples. Each peptide sample was separated using an Ultimate 3000 UPLC system (Thermo), under a 5-95% acetonitrile (ACN) gradient on a 15 cm × 100 µm Acclaim PepMap 100 C18 column (Thermo Scientific), at a flow rate of 300 µl/min over 1 hour. The Q Exactive plus (Thermo) mass spectrometer was operated in a full MS scan range of 150-2,000 m/z, a resolution of 70,000, and an AGC target value of 1 × 10⁶. Higher-energy collisional dissociation (HCD) was conducted under conditions of a fixed injection time of 120 ms, a resolution of 35,000 (m/z), and an HCD collision energy of 30%. Protein search was performed using the Homo sapiens (Uniprot 9606) protein database (DB) with the Proteome Discoverer software (ver. 2.5) by the identification & label-free quantitative (LFQ) method, and the results are shown in FIG. 33.

### Example 7.2 - Analysis of extracellular matrix self-assembly depending on presence or absence of matrix-bound nanovesicles in extracellular matrix

For scanning electron microscopy, a carbon tape was attached to a stub, on which the extracellular matrix sample was subsequently thinly spread, and then dried in a desiccator for at least 3 hours. Thereafter, the sample surface was coated with platinum using a sputter coater, followed by image analysis at an accelerating voltage of 5 kV and 6,000× magnification.

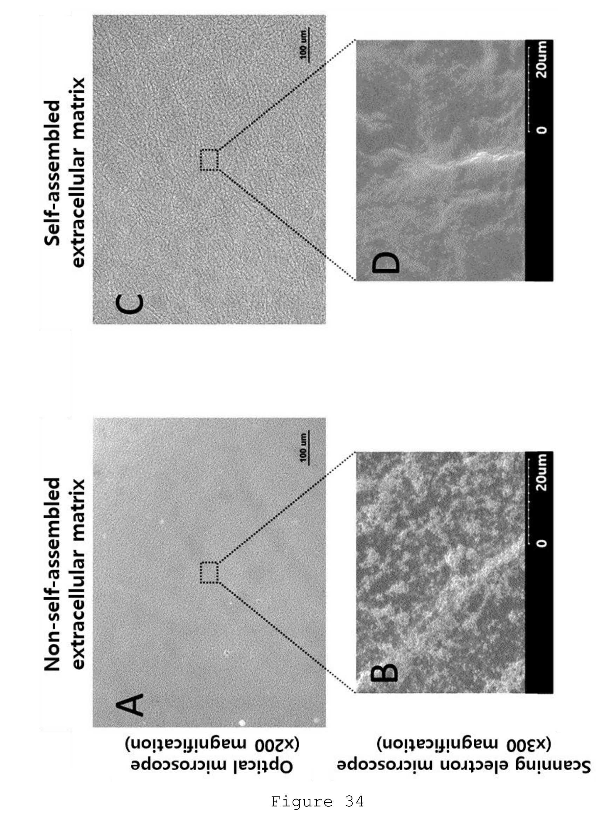

The results are shown FIG. 34.

When the matrix-bound nanovesicles were neither removed nor dissociated as shown in Panel B of FIG. 34, the extracellular matrix was not self-assembled into fibers as shown in Panel A of FIG. 34. However, when the matrix-bound nanovesicles were removed or dissociated as shown in Panel D of FIG. 34, the extracellular matrix self-assembled into fibers was observed in Panel C of FIG. 34. These results support that non-removed and non-dissociated matrix-bound nanovesicles inhibit self-assembly of the extracellular matrix.

Although the disclosure has been described in detail with reference to the specific features, it will be apparent to those skilled in the art that this description is only for a preferred embodiment and does not limit the scope of the disclosure.

## Claims

1. A composition for extracellular matrix production comprising an extracellular matrix and matrix-bound nanovesicles.

2. The composition of claim 1, wherein the composition is obtained by culturing connective tissue cells or connective tissue cells and epithelial tissue cells in a culture medium.

3. The composition of claim 2, wherein the culture medium is a serum-free culture medium.

4. The composition of claim 2, wherein the culture medium is a xeno-free culture medium.

5. The composition of claim 2, wherein the culture medium contains at least one selected from the group consisting of a glucocorticoid, insulin, and a growth factor.

6. The composition of claim 1, wherein the matrix-bound nanovesicles inhibit self-assembly of the extracellular matrix.

7. The composition of claim 6, wherein the self-assembly includes fibrillation, network forming, or a combination thereof of the extracellular matrix.

8. The composition of claim 1, wherein the extracellular matrix comprises at least one selected from the group consisting of laminin, collagen, fibronectin, proteoglycan, entactin, and fibulin.

9. A method for preparing a composition for extracellular matrix production containing an extracellular matrix and matrix-bound nanovesicles, the method comprising:
(a) culturing connective tissue cells in a culture medium containing at least one selected from the group consisting of a glucocorticoid, insulin, and a growth factor to obtain a culture or a cell layer; and
(b) collecting an extracellular matrix and matrix-bound nanovesicles from the culture or cell layer in step (a).

10. The method of claim 9, wherein the connective tissue cells are derived from humans.

11. The method of claim 9, wherein the connective tissue cells are at least one selected from the group consisting of fibroblasts, osteocytes, adipocytes, chondrocytes, ligament cells, tendon cells, mesenchymal stem cells, and cancer-associated fibroblasts.

12. The method of claim 9, wherein the extracellular matrix comprises at least one selected from the group consisting of laminin, collagen, fibronectin, proteoglycan, entactin, and fibulin.

13. The method of claim 9, wherein the culture medium is a serum-free culture medium.

14. The method of claim 9, wherein the culture medium is a xeno-free culture medium.

15. The method of claim 9, wherein the glucocorticoid is at least one selected from the group consisting of dexamethasone, hydrocortisone, prednisone, prednisolone, methylprednisolone, betamethasone, triamcinolone acetonide, fludrocortisones, and cortisol.

16. The method of claim 9, wherein the growth factor is at least one selected from the group consisting of ErbB signaling growth factor, fibroblast growth factor (FGF), keratinocyte growth factor (KGF), insulin-like growth factor (IGF), nerve growth factor (NGF), platelet-derived growth factor (PDGF), transforming growth factor-β (TGF-β), and vascular endothelial growth factor (VEGF).

17. The method of claim 16, wherein the ErbB signaling growth factor is at least one selected from the group consisting of epidermal growth factor (EGF), transforming growth factor-a (TGF-α), heregulin-β (HRG-β), heparin-binding EGF-like growth factor, amphiregulin, betacellulin, epiregulin, epigen, and neuregulins.

18. The method of claim 9, wherein the culture medium further contains at least one selected from the group consisting of ascorbate-2-phosphate, isoprenaline, and triiodothyronine.

19. The method of claim 9, wherein the culture is obtained upon exchange of the culture medium.

20. The method of claim 19, wherein the exchange of the culture medium is performed every 2 to 3 days.

21. The method of claim 9, wherein the cell layer is obtained after completion of culture.

22. The method of claim 9, wherein the culturing is performed using a culture containing an antioxidant.

23. The method of claim 22, wherein the antioxidant is at least one selected from the group consisting of vitamin A, vitamin C, vitamin E, selenium, coenzyme Q10, catechin, N-acetylcysteine (NAC), glutathione, beta-carotene, lycopene, lutein, polyphenols, cysteine, and taurine.

24. The method of claim 9, wherein the culturing of the cells is performed by at least one culture method selected from the group consisting of hypoxic culture, batch culture, fed-batch culture, and continuous culture.

25. The method of claim 9, wherein in step (a), the culturing is performed by addition of epithelial tissue cells.
